# EUROPÄISCHE PATENTANMELDUNG

(11) **EP 1 894 527 A2**
(43) Veröffentlichungstag der Anmeldung: **05.03.2008**
(21) Anmeldenummer: 07115494.2
(22) Anmeldetag: 03.09.2007
(51) Int. Cl.: A61B 6/03, A61B 19/00, A61B 6/00

(54) **Verfahren zur Unterstützung der Durchführung einer bildgestützten medizinischen Maßnahme und mit diesem Verfahren betriebene Einrichtung**

(30) Priorität: 04.09.2006 DE 102006041457
(71) Anmelder: SIEMENS AKTIENGESELLSCHAFT, 80333 München (DE)
(72) Erfinder: Fischer, Daniel, 91052 Erlangen (DE); Orman, Jasmina, 91052 Erlangen (DE)

(57) **Zusammenfassung**

Bei einem Verfahren und einer Einrichtung zur Unterstützung der Durchführung einer medizinischen Maßnahme an einem Patienten bei dem der Fortschritt der Maßnahme für den Patienten mit einer optischen Anzeige (20) sichtbar angezeigt wird. Dadurch wird die mit der Maßnahme einhergehende Belastung für den Patienten subjektiv verringert.

## Beschreibung

Die Erfindung betrifft ein Verfahren zur Unterstützung der Durchführung einer bildgestützten medizinischen Maßnahme an einem Patienten. Außerdem betrifft die Erfindung eine Einrichtung zur bildgestützten Durchführung einer medizinischen Maßnahme, die nach einem solchen Verfahren arbeitet.

Die Durchführung einer medizinischen Maßnahme ist in vielen Fällen für den Patienten mit einer erheblichen physischen oder psychischen Belastung verbunden. Ursache hierfür ist, dass sich der Patient in einer für ihn unangenehmen Situation während eines für ihn nur bedingt durchschaubaren Vorganges befindet. Der damit verbundene Stress kann unter Umständen zu einer verringerten Kooperationsbereitschaft des Patienten führen. Dies wiederum kann den Zeitbedarf für die medizinische Maßnahme zusätzlich erhöhen

Bei einer solchen medizinischen Maßnahme kann es sich beispielsweise um eine zeitintensive Röntgenuntersuchung im Rahmen einer Computertomographie oder in der Mammographie bei der Tomosynthese handeln, bei denen ein aus mehreren Schichtbildern aufgebauter 3D-Bilddatensatz (3D-Bild) aus einer Vielzahl einzelner 2D-Röntgenbilder rekonstruiert wird, oder es kann ein Eingriff, beispielsweise eine Biopsie, sein, der mit einem tomographischen oder tomosynthetischen Röntgenbild unterstützt wird. Besonders unangenehm ist die Situation für weibliche Patienten bei der Mammographie, bei der die Brust während der Röntgenaufnahmen zwischen einer Lagerplatte und einer Kompressionsplatte in einem komprimierten Zustand gehalten wird.

Der Erfindung liegt nun die Aufgabe zu Grunde, ein Verfahren zur Unterstützung der Durchführung einer bildgestützten medizinischen Maßnahme an einem Patienten anzugeben, mit dem die vom Patienten subjektiv empfundene Belastung verringert ist. Außerdem liegt der Erfindung die Aufgabe zu Grunde, eine mit diesem Verfahren betriebene Einrichtung anzugeben.

Hinsichtlich des Verfahrens wird die genannte Aufgabe gemäß der Erfindung gelöst mit einem Verfahren mit den Merkmalen des Patentanspruches 1. Gemäß der Erfindung wird bei einem solchen Verfahren die Durchführung einer bildgestützten Maßnahme an einem Patienten dadurch unterstützt, dass der Fortschritt der Maßnahme für den Patienten sichtbar optisch angezeigt wird.

Unter dem Begriff medizinische Maßnahme ist dabei im Sinne die vorliegenden Erfindung sowohl ein Vorgang zu verstehen, bei der lediglich ein oder eine Serie von Röntgenbildern aufgenommen werden, als auch ein Eingriff zu verstehen, der beispielsweise mit Hilfe von während der Maßnahme erzeugten Röntgenbildern stattfindet.

Die Erfindung beruht dabei auf der Erfahrung, dass das subjektive Empfinden des Patienten unabhängig von der tatsächlich objektiven Belastung durch äußere nicht unmittelbaren Zusammenhang mit der Maßnahme stehende Faktoren beeinflusst werden können. So wird die Zeitdauer einen unangenehmen Situation als weniger lang empfunden, wenn der Patient den Fortschritt der Maßnahme optisch verfolgen kann.

Das Verfahren gemäß der Erfindung ist deshalb insbesondere für Prozeduren geeignet, bei denen während der Maßnahme oder als Maßnahme eine Mehrzahl von 2D-Röntgenbildern mit unterschiedlichen Projektionswinkeln aufgenommen werden, aus denen ein 3D-Bilddatensatz, insbesondere bei der Mammographie ein tomosynthetisches 3D-Bilddatensatz, rekonstruiert wird.

Ein solcher positiver, für den Patienten subjektiv die Dauer der Maßnahme verkürzender Effekt wird insbesondere dann erzielt, wenn der Fortschritt der Maßnahme in Relation zu einer Gesamtdauer angezeigt wird. Dadurch erhält der Patient eine Vorstellung vom zeitlichen Ablauf, d.h. von der Geschwindigkeit, in der die Maßnahme durchgeführt wird, und erhält eine Vorstellung davon, wann die Maßnahme voraussichtlich beendet sein wird.

Dieses subjektive Zeitempfinden wird außerdem zusätzlich dadurch verkürzt, wenn die Restdauer der Maßnahme für den Patienten erkennbar angezeigt wird.

Eine solche optische Anzeige ist auch für das Bedienpersonal hilfreich und verkürzt auch für dieses subjektiv die Wartezeiten zwischen Beginn und dem Ende der Maßnahme.

In einer weiteren vorteilhaften Ausgestaltung der Erfindung wird der Fortschritt der Maßnahme zusätzlich akustisch angezeigt. Dies ist insbesondere für sehschwache Personen eine Hilfe.

Hinsichtlich der Einrichtung wird die genannte Aufgabe gelöst mit einer Einrichtung mit den Merkmalen des Patentanspruches 7, deren Vorteile ebenso wie die Vorteile der Ausgestaltungen gemäß der diesem zugeordneten Unteransprüche dem zu den Verfahrensansprüchen jeweils angegebenen Vorteilen sinngemäß entsprechen.

Zur weiteren Erläuterung der Erfindung wird auf das Ausführungsbeispiel der Zeichnung verwiesen. Es zeigen:
- Fig. 1: eine Einrichtung gemäß der Erfindung anhand eines tomosynthesefähigen Mammographiegerätes in einer schematischen Prinzipdarstellung,
- Fig. 2: ein Ausführungsbeispiel, in dem veranschaulicht wird, wie der Fortschritt der Maßnahme bei einem Mammographie-Verfahren in einer besonders vorteilhaften Ausgestaltung visualisiert wird.

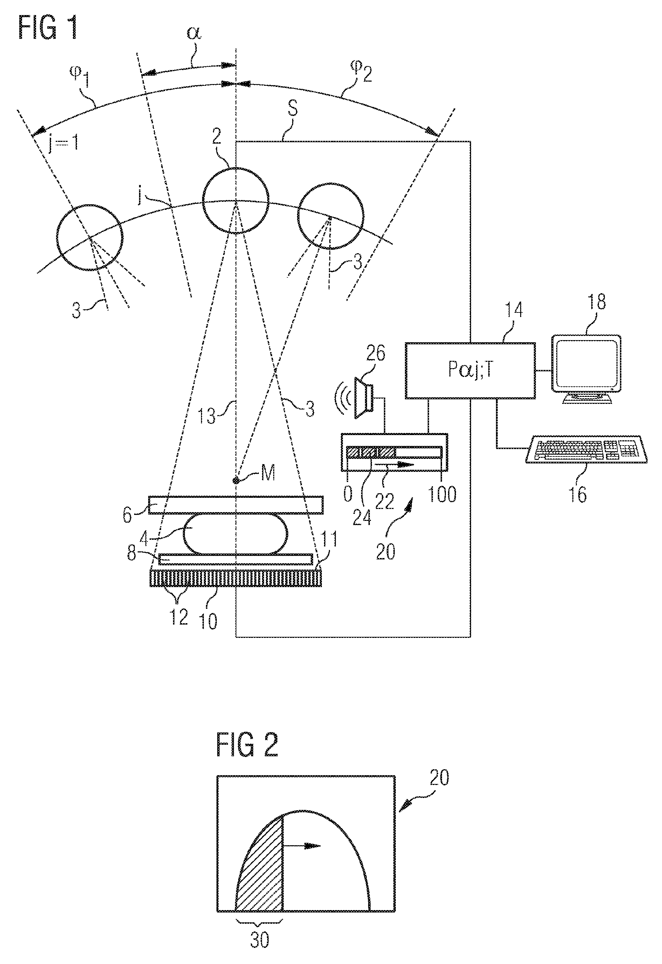

Gemäß Figur 1 umfasst die Einrichtung beispielsweise eine bildgebende Röntgenanlage, im Ausführungsbeispiel ein tomosynthesefähiges Mammographiegerät, eine Röntgenröhre 2 zum Erzeugen von Röntgenstrahlen 3, die ein Untersuchungsobjekt 4 durchqueren. Bei dem Untersuchungsobjekt 4 handelt es sich um eine weibliche Brust, die zwischen einer Kompressionsplatte 6 und einer Lagerplatte 8 eingebettet ist. Die das Untersuchungsobjekt 4, die Kompressionsplatte 6 und die Lagerplatte 8 durchquerenden Röntgenstrahlen 3 werden von einem großflächigen digitalen Röntgendetektor 10 empfangen, der aus einer Vielzahl von in einem matrixförmigen Array angeordneten Einzeldetektoren 12 aufgebaut ist, und dessen Empfangsfläche 11 parallel zu den Kompressionsplatten 6, 8 angeordnet ist.

Die Röntgenröhre 2 ist in einem begrenzten Bereich zum Untersuchungsobjekt ortsveränderbar angeordnet, und kann beispielsweise in einem begrenzten Winkelbereich ϕ₁, ϕ₂ um eine zur Zeichenebene senkrechte Achse M in unterschiedliche Winkelpositionen j=1 ... n geschwenkt werden, so dass vom Untersuchungsobjekt 4 mit verschiedenen Projektionswinkeln αⱼ relativ zur Normalen 13 der Empfangsfläche 11 des Röntgendetektors 10 Einzelbilder erzeugt werden können. Der Winkelbereich ϕ₁,ϕ₂ muss dabei nicht symmetrisch zur Normalen 13 angeordnet sein. Diese 2D-Röntgenbilder bzw. die diesen jeweils zugeordneten Projektionsdaten P_{αj} werden in einer einen Bildrechner enthaltenden Steuer- und Auswerteeinrichtung 14 durch Rekonstruktion zu einem aus mehreren Schichtbildern aufgebauten tomosynthetischen 3D-Bilddatensatz T zusammengesetzt und auf einem Monitor 18 dargestellt. Der Röntgendetektor 10 ist während der Schwenkbewegung der Röntgenröhre 2 ortsfest. Grundsätzlich ist es jedoch auch möglich, den Röntgendetektor 10 gemeinsam mit zu schwenken oder der Schwenkbewegung der Röntgenröhre 2 folgend linear zu verschieben.

Auch eine Bewegung der Röntgenröhre 2 auf einer begrenzten linearen Bahn statt des Schwenks ist zulässig, so dass die Höhendifferenz zwischen Röntgendetektor 10 und Röntgenröhre konstant bleibt. Diese lineare Bahn muss ebenfalls nicht notwendigerweise symmetrisch zur Normalen 13 verlaufen. Bei dieser linearen Bewegung erfolgt ein Ausrichten der Röntgenröhre 2 auf das Untersuchungsobjekt 4, so dass auch in diesem Fall vom Untersuchungsobjekt 4 Einzelbilder unter verschiedenen Projektionswinkeln αⱼ aber in einem begrenzten Winkelbereich aufgenommen werden.

Die Steuerung der Winkelposition j oder im Falle einer linearen Verschiebung der Linearposition und der Ausrichtung der Röntgenröhre 2 sowie ihrer Betriebsparameter erfolgt durch Steuersignale S, die von der Steuer- und Auswerteeinrichtung 14 generiert werden. Mit Hilfe von Eingabeelementen, im Beispiel sinnbildlich veranschaulicht durch eine Tastatur 16, können das Mammographiegerät gesteuert und unterschiedlich verschiedene Betriebsarten vom Bedienpersonal ausgewählt und durchgeführt werden.

Während der Aufnahme der für ein solches tomosynthetisches 3D-Röntgenbild T erforderlichen Vielzahl von 2D-Röntgenbildern (Einzelbilder oder Projektionsdaten P_{αj}) befindet sich die Patientin in einer für sie unangenehmen und durchaus auch schmerzhaften Situation. Um der Patientin zumindest subjektiv Entlastung zu verschaffen, ist in Sichtweite der Patientin eine optische Anzeige 20 angeordnet, mit der der Fortschritt der Maßnahme, im dargestellten Beispiel eine Information über den Fortgang der zum Erzeugen eines tomosynthetischen 3D-Bilddatensatzes T erforderlichen Prozedur - Schwenk der Röntgenröhre 2 in die unterschiedlichen Winkelpositionen j und jeweilige Erzeugung eines 2D-Röntgenbildes - angezeigt wird. Im Beispiel der Figur ist dies ein von links nach rechts in Richtung des Pfeils 22 wandernder Laufbalken 24, anhand dessen Laufgeschwindigkeit und der Länge der Reststrecke die Patientin eine Information über den Fortgang der Maßnahme und das voraussichtliche Ende erhält. Zusätzlich ist ein akustischer Signalgeber 26 vorgesehen, mit dem der Fortschritt der Maßnahme durch ein akustisches Signal, beispielsweise dann, wenn der Laufbalken 24 um ein Inkrement weiter springt, angezeigt wird.

Im dargestellten Ausführungsbeispiel ist die optische Anzeige 20 als separates, beispielsweise vom Bedienpersonal in Sichtweite der Patientin platzierbares Gerät dargestellt. Grundsätzlich ist es auch möglich, die optische Anzeige in die Einrichtung, z. B. in die Bildschirmoberfläche des zur Bildwiedergabe vorgesehenen Monitors 18 zu integrieren.

Anstelle des dargestellten Lauf- oder Progressbalkens sind auch andere geometrische Darstellungsformen denkbar, beispielsweise ein Progressring, ein Progresskreis oder ein Progressdreieck. Die Fortschrittsanzeige kann auch mit Hilfe einer Mehrzahl nebeneinander angeordneter Leuchtdioden erfolgen. Ebenso kann der Fortschritt der medizinischen Maßnahme durch Zahlen beispielsweise zwischen 0 und 100% oder durch zahlenmäßige Angabe der Restzeit visualisiert werden.

Eine weitere Möglichkeit der optischen Darstellung des Fortschritts der Maßnahme ist in Fig. 2 dargestellt. Dort ist die optische Anzeige 20 für den Fortschritt durch einen Füllgrad 30 einer in Brustform auf einem Monitorbild wiedergegebenen Fläche visualisiert.

## Patentansprüche

1. Verfahren zur Unterstützung der Durchführung einer medizinischen Maßnahme an einem Patienten, bei dem der Fortschritt der Maßnahme für den Patienten sichtbar optisch angezeigt wird.

2. Verfahren nach Anspruch 1, bei dem der zeitliche Fortschritt der Maßnahme in Relation zu einer Gesamtdauer angezeigt wird.

3. Verfahren nach Anspruch 1 oder 2, bei dem eine Restdauer der Maßnahme angezeigt wird.

4. Verfahren nach einem der vorhergehenden Ansprüche, bei dem der Fortschritt akustisch wiedergegeben wird.

5. Verfahren nach einem der vorhergehenden Ansprüche, bei dem während der Maßnahme oder als Maßnahme eine Mehrzahl von 2D-Röntgenbildern (Pαⱼ) mit unterschiedlichen Projektionswinkeln (αⱼ) aufgenommen wird, aus denen ein 3D-Bilddatensatz (T) rekonstruiert wird.

6. Verfahren nach Anspruch 5, bei dem ein tomosynthetischer 3D-Bilddatensatz (T) erzeugt wird.

7. Einrichtung zur bildgestützten Durchführung einer medizinischen Maßnahme an einem Patienten, mit einer optischen Anzeige (20) zum für den Patienten sichtbaren Anzeigen des Fortschritts der Maßnahme.

8. Einrichtung nach Anspruch 7, mit einem akustischen Signalgeber (26) zum Erzeugen eines den Fortschritt anzeigenden akustischen Signals.

9. Einrichtung nach Anspruch 7 oder 8, mit einer bildgebenden Röntgenanlage zum Erzeugen eines aus einer Mehrzahl von mit unterschiedlichen Projektionswinkeln (αⱼ) aufgenommenen 2D-Röntgenbildern (Pαⱼ) rekonstruierten 3D-Röntgenbildes (T).

10. Einrichtung nach Anspruch 9, bei der die bildgebende Röntgenanlage ein Mammographiegerät zum Erzeugen eines tomosynthetischen 3D-Röntgenbildes (4) ist.
